# EUROPEAN PATENT APPLICATION

(11) **EP 1 386 592 A2**
(43) Date of publication of application: **04.02.2004**
(21) Application number: 03078215.5
(22) Date of filing: 26.07.1995
(51) Int. Cl.: A61F 5/448, A61L 24/00

(54) **Resealable coupling device**

(30) Priority: 26.07.1994 US 280428
(62) Divisional of application: 95927442.4
(71) Applicant: E.R. SQUIBB & SONS, INC., Princeton, New Jersey 08543-4000 (US)
(72) Inventor: The designation of the inventor has not yet been filed
(74) Representative: Plougmann & Vingtoft A/S

(57) **Abstract**

There is described a resealable coupling device for use in medical applications. The device includes a pair of first and second coupling members (4, 6). The first coupling member (4) has a first opposed surface for releasably engaging a second opposed surface of the second coupling member to form a releasable fluid-tight seal therewith. The device is distinguished in that at least one of respective opposed surfaces of said first (4) and second (6) coupling members having thereon a single layer of a water-washable adhesive operable to provide releasable and resealable engagement of said first and second coupling members.

There is also provided a method of releasably coupling first and second components of a medical device together. The method is distinguished in that includes the step of reversibly engaging the first and second components through a single layer of a water-washable adhesive to provide a passageway for the flow of fluid through the device.

There is also provided an adhesive for coupling first and second opposing surfaces in a medical device. The adhesive comprises at least one thermoplastic elastomer. Preferably, the thermoplastic elastomer is a styrene copolymer or an acrylic resin.

## Description

### Field of the invention

The present invention is directed to the field of resealable coupling devices comprising associated coupling members releasable secured to each other through a washable adhesive composition, the coupling devices being suitable for use in medical applications. Moreover, the invention is also directed towards methods of releasably securing coupling members to each other through a washable adhesive composition. Furthermore, the present invention is also directed towards washable adhesive compositions, for example for use in releasable securing coupling members together as in devices adapted for medical applications.

### Background to the invention

Medical drainage devices and appliances, for example ostomy appliances, generally each include an associated bag or pouch for collecting bodily wastes discharged from their surgically created stoma, namely surgically-created hole. The bag or pouch is connected to a pad or surgical dressing which is in contact with a patient's skin and surrounds the stoma.

Most such appliances employ a coupling between the bag and the dressing which enables the bag to be readily removed when necessary, and replaced by a clean, empty bag. The coupling should provide a fluid tight seal to prevent leakage of liquids and/or gases.

Coupling system are known in the art for reversibly securing principal components of an appliance to each other. Such systems are disclosed in earlier literature, for example:
(a) in United States patent nos. US 4, 232, 672, US 4, 460, 363 and US 4, 518, 389, US associated with Peter L. Steer et al.;
(b) in United States patent nos. US 4, 636, 205, US 4, 710, 183 and US 4, 701, 169 associated with Peter L. Steer;
(c) in United States patent no. US 4, 648, 875 associated with Keith T. Ferguson; and
(d) in United States patent no. US 4, 642, 107 associated with Ronald Arnone,
each of these United States patents being incorporated herein by reference.

Each of these systems relies on a mechanical coupling system to secure its bag to a dressing surrounding a stoma. Mechanical coupling devices, however, are prone to leakage because contacting faces thereof eventually move out of face-to-face alignment. Such leakage occurs as a result of prolonged use of the device, changes in temperature and from manufacturing defects. It should be noted that as the diameter of the mechanical coupling device increases, the security of the device generally decreases. Specifically, the larger diameter devices are more susceptible to being dislodged when the patient moves.

Ostomy appliances generally adhere to a patient's skin through the use of an adhesive. Such an adhesive is typically pressure sensitive and may contain additives which have soothing and/or healing properties to minimize patient discomfort during use of the ostomy appliance. For example, in United States patent no. US 4, 253, 460 associated with James L. Chen, this patent being incorporated herein by reference, discloses an adhesive composition composed of a hydrocolloid agent, a pressure sensitive adhesive and a cohesive strengthening agent, optionally including pectin and gum karaya. Other examples of adhesive compositions for this use are disclosed:
(a) in United States patent no. US 4, 551, 490 associated with Arthur Doyle et al.;
(b) in United States patent no. US 4, 393, 080 associated with John M. Pawelchak et al.,
these patents each being incorporated by reference. Included amongst the disclosed adhesive compositions is a homogenous blend of mineral oil, at least one polyisobutylene alone or with an elastomer such as a styrene radical or block type copolymers. The styrene copolymers include commercially available products sold by Shell Chemical Company under the trade name "Kraton".

In a United States patent no. US 4, 894, 058 associated with Jensen, there is disclosed an ostomy device having an adhesive attachment system for multiple use. The system provides for a face plate with a surface flange, to which is mounted a plurality of adhesive connecting angular parts or rings, each having a covering layer. Each ring is provided with a radially extended gripping portion to permit a user thereof to grip each ring in turn, for example for removal purposes. The rings are angularly offset such that each of the gripping portions is exposed, allowing the rings to be peeled off, one at a time. In such a manner, a pouch of the device can be repeatedly reattached, up to five to seven times, to the face plate by removing each ring in succession until all of the rings have been removed.

More recently, an ostomy appliance has been sold under a trade mark "Microskin" by Cymed, Inc. of Hayward, California. This appliance employs an adhesive to join facing surfaces of respective coupling elements. The coupling components are separated from each other by disrupting their adhesive seal and then attaching a new coupling device including a fresh ostomy bag. When the seal is broken, the respective adhesive surfaces often have attached thereto particles of waste products that must be removed before a new ostomy bag can be added. After cleaning, respective surfaces must be substantially free of all contaminants to prevent infection.

Conventional adhesives such as employed in the Microskin product are disadvantageous. Although such adhesives may be washable, they have an adhesive quality in which the adhesive sticks to patient preferred cleaning implements employed by the patient to clean the coupling surfaces such as tissue, cloths and the like. The conventional adhesive compositions typically retain small particles of fibres from such cleaning implements and therefore cannot provide a contamination free surface for attachment of the next ostomy bag. In addition, such products employ a silicon release paper which is difficult to separate from the adhesive layer, especially for elderly patients.

In order to avoid this problem, such prior art devices require organic solvents to clean the coupling surfaces and must be dried without the convenience of using patient preferred cleaning implements.

Applicant has appreciated that adhesive compositions can be employed to couple the principal components of an ostomy appliance together so as to eliminate the need for mechanical coupling devices. Such devices can provide a fluid tight seal that may be unsealed to replace the used ostomy bag with a new one and then resealed in fluid tight fashion. The problem of leakage of liquids and/or gases characteristic of ostomy appliances relying on mechanical seals can therefore be eliminated or at least minimized by using appropriate adhesive according to the present invention.

In addition, the present invention provides an adhesive coupling system that can provide a clean surface for the reapplication of a new ostomy bag using conventional cleaning implements.

### Summary of the invention

A first object of the invention is to provide an adhesive coupling device for containing liquids and/or gases, the device arranged to be devoid of mechanical couplings and thereby providing greater security against escape of the liquids and/or gases.

A second object of the invention is to provide an adhesive coupling device for containing liquids and/or gases, the device arranged to be readily cleanable to provide at least a substantially contamination free surface for application of receptacles, for example a new ostomy bag.

A third object of the invention is to provide one or more adhesives suitable for use in adhesively-coupling devices for containing liquids and/or gases.

According to a first aspect of the present invention, there is provided a resealable coupling device for use in medical applications, the device including a pair of first and second coupling members, said first coupling member having a first opposed surface for releasably engaging a second opposed surface of the second coupling member to form a releasable fluid-tight seal therewith,
characterized in that
at least one of respective opposed surfaces of said first and second coupling members having thereon a single layer of a water-washable adhesive operable to provide releasable and resealable engagement of said first and second coupling members.

The invention is of advantage in that it is capable of addressing at least one of the objects of the invention.

Preferably, in the device, said adhesive layer is included on each of said opposed surfaces.

Preferably, in the device, the adhesive includes at least one thermoplastic elastomer. More preferably, the thermoplastic elastomer is a styrene copolymer or an acrylic resin. Moreover, the styrene copolymer is preferably selected from a group consisting of strene-acrylonitrile-butadiene copolymer having a relatively high butadiene content.

Preferably, in the device, the styrene copolymer is a styrene-isoprene-styrene copolymer or a styrene-acrylonitrile-butadiene copolymer having a relatively high butadiene content. Moreover, the styrene copolymer is preferably combined with an oil to form a gel.

Preferably, in the device, the adhesive comprises an elastomeric composition having a modulus of elasticity sufficient to enable waste particles to be readily removed therefrom, and having an internal viscosity low enough to enable water-washing an high enough to provide tack. More preferably, the modulus of elasticity is in a range of about 1 to 100 psi and the internal viscosity is in a range of about 1000 to 20, 000 poises.

Preferably, in the device, the adhesive further comprises a plasticizer.

Preferably, in the device, the adhesive is solid, hydrophobic and insoluble in water.

Preferably, in the device, the adhesive comprises high molecular weight polyvinyl chloride.

Preferably, in the device, the adhesive comprises a copolymer of vinyl chloride and vinyl acetate.

Preferably, in the device, the single layer of the washable adhesive is on one of said single opposed second surfaces.

Preferably, in the device, at least one of the opposed surfaces has an irregular surface to facilitate anchoring of the adhesive.

Preferably, in the device, at least one of the opposed surfaces is comprised of a film or web. More preferably, said film or web is made of a non-woven fabric. Most preferably, the non-woven fabric is made of a material selected from a group consisting of polypropylene, polyethylene, polyester, rayon and blends thereof.

According to a second aspect of the present invention, there is provided a method of releasably coupling first and second components of a medical device together, characterized in that the method includes the step of:
reversibly engaging the first and second components through a single layer of a water-washable adhesive to provide a passageway for the flow of fluid through the device.

Preferably, in the method, the adhesive includes at least one thermoplastic polymer. More preferably, the thermoplastic elastomer is a styrene copolymer or an acrylic resin. Most preferably, the styrene copolymer is selected from a group consisting of styrene-acrylonitrile-styrene, styrene-butadiene-styrene, styrene-isoprene-styrene and styrene-ethylene/butylene-styrene.

Preferably, to render the method more effective, the adhesive has a modulus of elasticity sufficient to enable waste particles to be removed therefrom and has an internal viscosity low enough to enable water-washing and high enough to provide tack. More preferably, the modulus of elasticity is in a range of 1 to 100 psi and the internal viscosity is in a range of about 1000 to 20, 000 poises.

Preferably, in the method, the adhesive further comprises a plasticizer.

Preferably, in the method, the adhesive is solid, hydrophobic and insoluble in water.

Preferably, in the method, the adhesive comprises high molecular weight polyvinyl chloride. Alternatively, the adhesive comprises a copolymer of vinyl chloride and vinyl acetate.

According to a third aspect of the present invention, there is provided an adhesive for coupling first and second opposing surfaces in a medical device, said adhesive comprising at least one thermoplastic elastomer.

Preferably, in the adhesive, the thermoplastic elastomer includes a styrene copolymer or an acrylic resin.

Preferably, in the adhesive, the styrene copolymer is selected from a group consisting of strene-acrylonitrile-butadiene copolymer having a relatively high butadiene content. More preferably, the styrene copolymer is a styrene-isoprene-styrene copolymer or a styrene-acrylonitrile-butadiene copolymer having a relatively high butadiene content.

Preferably, in the adhesive, the styrene copolymer is combined with an oil to form a gel.

Preferably, the adhesive comprises an elastomeric composition having a modulus of elasticity sufficient to enable waste particles to be readily removed therefrom, and having an internal viscosity low enough to enable water-washing an high enough to provide tack.

Preferably, for the adhesive, the modulus of elasticity is in a range of about 1 to 100 psi and the internal viscosity is in a range of about 1000 to 20, 000 poises.

Preferably, the adhesive further comprises a plasticizer.

Preferably, the adhesive is solid, hydrophobic and insoluble in water.

Preferably, the adhesive comprises high molecular weight polyvinyl chloride.

Preferably, the adhesive comprises a copolymer of vinyl chloride and vinyl acetate.

### Brief description of the drawings

The following drawings are illustrative of embodiments of the invention and are not intended to limit the invention as encompassed by the claims forming part of the application:
- Figure 1: is an exploded view of an ostomy appliance utilizing the present invention;
- Figure 2A: is a side view of an embodiment of the invention utilizing an adhesive on a receptacle side component only; and
- Figure 2B: is a side view of a further embodiment of the invention with an adhesive on a bodyside component only.

### Detailed description of embodiment of the invention

The present invention is directed to coupling members releasably secured to each other in which principal components thereof are coupled together using an adhesive in contradistinction to mechanical coupling devices characteristic of the prior art; such coupling members are especially suitable for use in ostomy appliances and wound drainage devices, although are susceptible to being used in other applications. Coupling with an adhesive provides a fluid-tight seal which can be peeled apart and then subsequently resealed. The present invention ensures substantially leak proof operation, for example of the ostomy appliance, during repeated use. The preferred adhesives according to the invention are those that can be readily washed by conventional cleaning implements such as tissues, cloths and the like and still provide a substantially contamination free surface, especially free of fibres and the like.

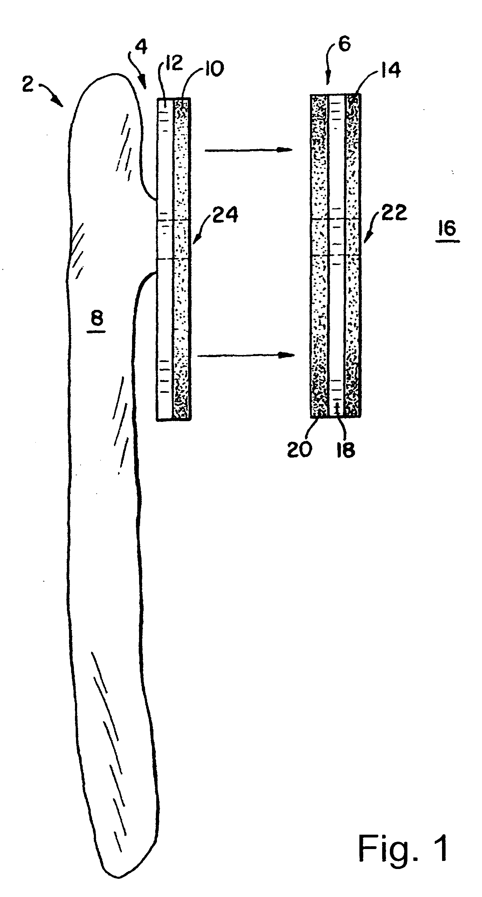

Referring to the drawings and firstly to Figure 1, there is shown an ostomy appliance 2 having two components capable of being coupled together in a fluid tight relationship. The first of these components is a receptacle side component 4 and the second component is a bodyside component 6. The receptacle side component 4 includes a receptacle 8 for collecting discharged waste liquids and/or gases from a patient. As used herein, the term "receptacle" shall include bags, pouches and the like which can be employed to collect waste fluids from the ostomy patient.

In accordance with the present invention, the receptacle side component 4 includes an adhesive layer 10 connected to the receptacle 8 through a substrate 12 which provides support for the adhesive layer 10. The substrate 12 can be made from any material which provides a surface for placement of the adhesive. Preferred substrates are webs or films, preferably having an irregular surface, for example naps, to ensure anchoring of the adhesive 10 thereto. Examples of such substrates include non-woven fabrics made of polypropylenes, polyethylenes, polyesters, rayons and the like including blends thereof. Preferably, the adhesive is impregnated into the substrate.

The adhesives which are used to form the adhesive layer 10 include thermoplastic elastomers such as styrene copolymers and acrylic adhesives. The preferred styrene copolymers include styrene-acrylonitrile-butadiene, styrene-butadiene-styrene, styrene-isoprene-styrene, styrene-ethylene/butylene-styrene and the like and blends thereof. Such copolymers of styrene are available, for example from Shell Chemical Company under a trade mark "Kraton". A preferred styrene copolymer is styrene-isoprene-styrene copolymer, for example Kraton D 1107 from Shell Chemical Company. Another preferred copolymer is styrene-acrylonitrile-butadiene having a relatively high butadiene content.

A preferred adhesive is one which may be washed and reused. Washable adhesives of this type include a gelled " Kraton" adhesive, for example Kraton D 1107, in which the styrene based polymer is gelled with an oil, preferably mineral oil. The amount of the styrene based polymer is preferably from about 70% to 90% by weight and the amount of the mineral oil is from about 10% to 30% by weight, based on the total weight of the styrene based polymer.

The adhesive composition of the type described above may be prepared, for example, by heating the mineral oil to a temperature of from about 120 °C to 150 °C and then adding the copolymer, for example Kraton D 1107, under stirring until the copolymer is dissolved. The resulting adhesive composition can be poured onto a suitable substrate or applied by a common hot melt applicator.

The most preferred types of adhesive are those which can be washed by conventional cleaning implements such as tissues and cloths without retaining fibres thereof and which form a fluid-tight seal even after many applications. Such adhesives are disclosed, for example in a United States patent no. US 3, 682, 690 associated with Homer C. Amos et al., the patent being incorporated herein by reference.

In particular, the most preferred adhesive compositions are comprised of an elastomeric composition having a modulus of elasticity sufficient to enable waste particles to be readily removed therefrom with an internal viscosity low enough to enable water-washing and high enough to provide tack. Preferably, the modulus of elasticity is in the range of from about 1 to 100 psi and the internal viscosity is from about 1000 to 20, 000 poises. Preferred adhesive compositions are made of high molecular weight polyvinyl chloride or copolymers of vinyl chloride and vinyl acetate.

For water-washing applications as desired for the aforementioned ostomy appliance, the adhesive composition is hydrophobic wherein water will readily run off the surface, leaving the surface dry.

The softness of the tacky material used in the preferred adhesive composition can range from a modulus as low as 1, at which point the material is weak, to a modulus as high as 100. Values in the upper part of the range are satisfactory only with materials with a high "intrinsic adhesivity", namely a high surface free energy. In general, the softer, namely the lower the modulus, the greater the tack. Some materials, such as some polyvinyl chloride compositions, have a quite non-linear stress-strain curve. A first portion of the curve shows a very low modulus, but later the curve becomes very steep, indicating a very high modulus. In such materials, one can achieve great softness with relatively high toughness.

Another requirement for washable tackiness is particularly significant, is an internal viscosity between 1, 000 and 20, 000 poises.

Most elastomers require a plasticizer to achieve a modulus as low as is desired. Materials such as neoprene and high-molecular-weight vinyls have little internal viscosity of their own, and the viscosity of the plasticized material is a fairly accurate reflection of the viscosity of the plasticizer itself. In any event, the internal viscosity must be low enough so that the material can quickly flow into large surface contact with the surface of the object to which tack is sought. But the viscosity must also be high enough so that the material does not yield too quickly to any force seeking to remove the object tacked onto the adhesive. Too low a viscosity results in little apparent tack. On the other hand, too high a viscosity results in the material feeling tacky with sustained contact pressure but not flowing quickly enough into large surface contact, with the contact surface, which involves very short-time contact pressure. Experience has shown that a viscosity in the neighbourhood of 2500 to 5000 poises is most preferred. In general, viscosities outside the range of from 1000 to 20, 000 poises give inferior results.

The internal viscosities can be measured by conventional means. In the case of highly plasticized vinyls or neoprene, measurement is unnecessary, for the plasticizer used therewith determines the resultant viscosity. The internal viscosity of materials such as polysulfides or polyurethanes which do not employ a plasticizer can be determined by comparing them with materials whose viscosity is known from its plasticizer.

A ball of a material in question is prepared, and a similar ball is prepared of the same modulus in a mixture of a high-molecular weight polyvinyl chloride, such as Geon 121 with a plasticizer whose internal viscosity is known. The surfaces are dusted with talc or a similar powder, and rebounds of the balls are compared, the greater the rebound, the lower the viscosity. This method is crude but effective so long as care is taken to make the modulus, an easily measured property, of the two balls equal. A better method is that further described in a United States patent no. US 3, 682, 690.

Tack is the result of a particular degree of softness and internal viscosity in combination with a property which might be termed the "intrinsic adhesivity", but which is better known as "surface free energy", a degree to which Van der Waals forces within the material are bound. For example, in materials such as waxes or polytetrafluoroethylene, or in general materials composed of long unbranched chain molecules, the molecular bonds are tightly bound and show little of this quasi-chemical activity at the surface, and can be described as having a low intrinsic adhesivity. On the other hand, materials composed of short chain or highly branched molecules have many chain ends on any given surface, and thus have a high degree of quasi-chemical activity at the surface; these can be described as having a high intrinsic adhesivity. When compounding or choosing a washable tacky elastomer for a given application, a proper choice of characteristic as taught herein can be used to great advantage. For instance, when extreme ease of washability is desired, a material such as a very high molecular weight polyvinyl chloride can be used as a base resin. This is because of its low surface free energy and high elasticity will wash clean very easily, while the application of the principles taught herein regarding modulus and internal viscosity will give an aggressive tack even though the base resin has a low intrinsic adhesivity, by the addition of a suitable plasticizer.

If the plasticizer is used, it is important that it be highly compatible and not subject to excessive "sweating". It is preferred that the plasticizer be highly resistant to extraction by soapy water, since, otherwise, successive washings would soon destroy the efficacy of the material. The plasticizer should not be fugitive, namely it should have an extremely low vapour pressure, for example below 10⁻⁹ microns Hg. When used, the plasticizer imparts to the final product a desired value of internal viscosity and softness not inherent in those elastomers with which the plasticizer is used. If a copolymer of vinyl chloride and vinyl acetate is used, somewhat less plasticizer is preferred.

Almost any non-water-soluble elastomer will give satisfactory results if formulated in a manner described herein. High-molecular weight vinyl chloride plastics have advantages of low cost, ease of handling, transparency, low surface free energy, and a non-linear stress-strain curve lending toughness.

The adhesive composition may also include in addition to tackifiers, antioxidants, antibiotics, antimicrobial agents and the like in effective amounts known to those of ordinary skill in the art.

The bodyside component 6 of the ostomy appliance 2 includes a bodyside adhesive layer 14 which is adapted to adhere to the patient's skin and is shown generally by a numeral 16. Adhesive compositions which can be used to adhere the ostomy appliance 2 to the patient are known and include those previously mentioned in connection with United States patent nos. US 4, 253, 460, US 4, 393, 080 and US 4, 551, 490.

As shown in the embodiment of Figure 1, the bodyside component 6 is further provided with a substrate 18 and an adhesive layer 20 which correspond in structure and function to the substrate 12 and the adhesive layer 10, respectively of the receptacle side component 4. The substrate 18 is preferably a web or film made from non-woven fabrics including polypropylenes, polyethylenes, polyesters, rayons and the like and blends thereof. The web or film preferably has a nap or irregular surface which facilitates the anchoring of the adhesive thereto. The adhesive layer 20 is preferably made of the adhesives described above and in particular those mentioned in United States patent no. US 3, 682, 690. The adhesive employed for the adhesive layer 20 is washable and reusable so that the receptacle side component 4 and the bodyside component 6 may be peeled away from each other and a new receptacle inserted into the ostomy appliance with a substantially combination free coupling.

In operation, the bodyside component 6 of the ostomy appliance 2 is adhered to the patient's skin 16 by the adhesive layer 14 so that a passageway 22 therein is aligned with the stoma (not shown) created by the surgical procedure. The receptacle side component 4 having a similar passageway 24 is then placed into contact with the bodyside component 6 through the respective adhesive layers 10, 20 so that the respective passageways 22, 24 are aligned thereby enabling waste liquids and gases escaping from the stoma to proceed into the receptacle 8. When the receptacle 8 is filled with waste fluids or solids and must be replaced, the adhesive layer 10 is peeled away from the adhesive layer 20 thereby separating the receptacle side component 4 form the bodyside component 6 while the latter remains attached to the patient's skin.

Alternatively, the ostomy appliance may be removed from the patient's skin and then the components 4, 6 peeled away from each other. Once separated, the adhesive layer 20 is washed, preferably in a manner most suitable for the patient such as being water washed in tissues, cloth and the like. Washing provides a substantial contamination-free surface which is free of fibres and the like. The components 4, 6 are then reattached to adhesively reseal a new receptacle side component 4 through its adhesive layer 10. The ostomy appliance 2 is then ready for continued operation.

It should be understood that the disengagement of the components 4, 6 can be made at any time during operation of the ostomy device. For example, if an adjustment of the ostomy appliance 2 on the patient's skin is necessary, the receptacle side component 4 may first be peeled away from the bodyside component 6 as explained previously. The bodyside component 6 may then be removed and repositioned about the stoma followed by the resealing of the components 4, 6 through the respective adhesive layers 10, 20.

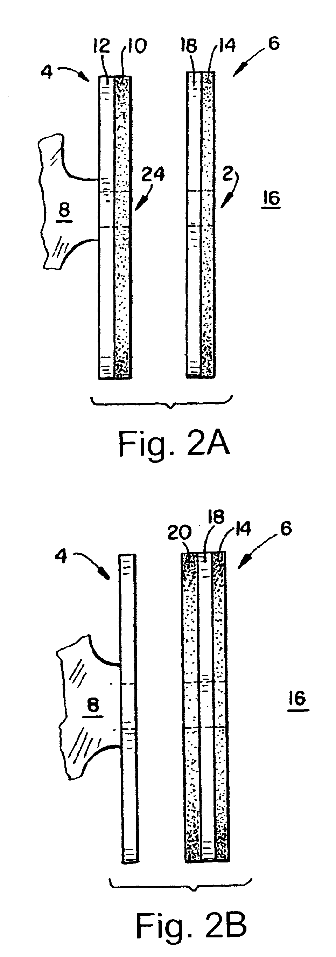

Although preferred particularly when using the preferred adhesive composition, it is not essential that both the receptacle side component 4 and bodyside component 6 have an adhesive layer. As shown in Figures 2A and 2B, only one of the components 4, 6 has an adhesive layer. More specifically, Figure 2A shows an embodiment of the ostomy appliance wherein the receptacle side component 4 has an adhesive layer 10 but the bodyside component 6 has been constructed without a corresponding adhesive layer.

The operation of the ostomy appliance shown in Figure 2A is essentially the same as that described above for the embodiment of Figure 1. The bodyside component 6 is adhered to the patient's skin and either before or after the procedure, the components 4, 6 are placed into contact wherein the adhesive layer 10 sealingly engages the substrate 18 of the bodyside component 6.

In a further embodiment of the invention as shown in Figure 2B, the bodyside component 6 is provided with an adhesive layer 20 but the receptacle side component is not provided with a corresponding adhesive layer. The components 4, 6, are placed in operative contact in the same manner as described above for the embodiment of Figure 2B.

As shown in the embodiments described above, the ostomy appliance employs a resealable adhesive based coupling mechanism that provides a fluid-tight seal and provides significant advantages over mechanical coupling mechanisms that are prone to leakage, particularly when subjected to repeated use. In addition, adhesive compositions employed in the present invention provide a surface which can be readily cleaned by conventional cleaning implements without leaving residual fibre materials thereon.

Reference signs used in the appended claims should not be seen as limiting the extent of the matter protected by the claims; their sole function is to make the claims easier to understand.

### EXAMPLE 1:

| Ingredient: | Parts by weight: |
|---|---|
| High-molecular-weight polyvinyl chloride, for example Geon 121 made by B. F. Goodrich | 100 |
| Plasticizer, a polyester condensation product of sebacic acid and ethylene glycol of approximately 8000 molecular weight, for example Paraplex G25 made by Rohm and Haas | 400 |
| Barium zinc phenate, for example Argus Chemical Co., Mark KCB | 4 |

Suitable pigment or dye, if desired, may be added. The suggested cure cycle is 10 minutes at 380 °F.

This composition has an internal viscosity of 2200 poises, a Young's modulus of about 10 p.s.i., and a permanent tack angle of 3°.

Similar results may be obtained by substituting a copolymer of polyvinyl chloride and polyvinyl acetate for the polyvinyl chloride, the copolymer preferably having approximately the same physical qualities as that of this example and usually requiring slightly less plasticizer.

### EXAMPLE 2:

| Ingredient: | Parts by weight: |
|---|---|
| High-molecular-weight polyvinyl chloride, for example Geon 121 made by B. F. Goodrich | 100 |
| Plasticizer, a polyester condensation product of sebacic acid and ethylene glycol of approximately 8000 molecular weight, for example Paraplex G25 made by Rohm and Haas | 400 |
| A glycerol ester of hydrogenated rosin, for example Staybelite Ester 10 made by Hercules, Inc. | 100 |
| Barium zinc phenate, for example Argus Chemical Co., Mark KCB | 4 |

Suitable pigment of dye, if desired, may be added. The suggested cure cycle is 10 minutes at 380 °F.

This composition has an internal viscosity of about 8 minutes, for example about 6, 000 poises. Moreover, it has a Young's modulus of about 10 p.s.i., and a permanent tack angle of about 3°.

### EXAMPLE 3:

The procedure of Example 1 is repeated except that 20% of the Geon 121 polyvinyl chloride is replaced by Geon 222, a very short chain polyvinyl chloride copolymer of high intrinsic adhesivity. The resulting composition has a tack similar to Example 1 but a higher intrinsic adhesivity or surface free energy.

### EXAMPLE 4:

The procedure of Example 1 is repeated except that 100 parts by weight of butyl benzyl phthalate is added. The resulting composition has an internal viscosity of about 1000 poises with reduced tack and modulus.

### EXAMPLE 5:

| Component A: ingredients: | Parts by weight: |
|---|---|
| Polyether triol, for example Wyandotte TP 4542 | 100 |
| Toluene di-isocyanate, for example Allied Chemical Nacconate | 12 |

The component A mixture is held at 160 °F for 4 hours.

| Component B: ingredients: | Parts by weight: |
|---|---|
| Polyether triol as in component A | 100 |
| Tin octoate catalyst, for example Witco Chemical Co. tin catalyst C-4 | 0.5 |

The two prepared components A and B are mixed in the proportions by weight of:
- Component A:: 100
- Component B:: 83

The mixture is then cured for 4 hours at 180 °F.

### EXAMPLE 6:

| Component A (resin component): ingredients: | Parts by weight: |
|---|---|
| Polyalkylene polysulfide, for example Thiokol LP 31 | 100 |
| Chlorinated biphenyl, for example Monsanto Aroclor 1254 | 90 |
| Calcium carbonate, for example Diamond Alkali Co. Super Multifex | 30 |
| Liquid coumarin-indene alkylated phenol, for example Neville Chemical Co. 10° Nevillac | 20 |
| Bis-phenol-A and epichlorhydrin epoxy resin, for example Shell Chemical Epon 836 | 3 |
| Stearic acid | 0.5 |
| Sulfur | 0.1 |

| Component B (catalyst component): ingredients: | Parts by weight: |
|---|---|
| Lead oxide | 100 |
| Chlorinated biphenyl, for example Monsanto Aroclor 1254 | 30 |
| Xylene | 10 |
| Zinc stearate | 2.5 |
| Stearic acid | 1.5 |

These two components A and B are mixed in a preferred ratio of one hundred parts by weight of the resin mix (component A) to three and one-half parts of the catalyst mix (component B) and then cured at 160 °F for two hours.

### EXAMPLE 7:

| Rubber solution: ingredients: | Parts by weight: |
|---|---|
| Polychloroprene, for example DuPont Neoprene W | 100 |
| Toluene | 240 |
| Methyl ethyl ketone | 160 |

The rubber solution mixture is stirred or tumbled until tne neoprene is dissolved.

| Premix of catalyst and plasticizers: | Parts by weight: |
|---|---|
| Chlorinated biphenyl, for example Monsanto Aroclor 1254 | 20 |
| Zinc oxide | 10 |
| Magnesium oxide | 5 |
| Phenyl-beta-naphthylamine, for example DuPont Neozone D | 2 |
| Ethyl thiourea, for example DuPont Accelerator NA22 | 1 |

The materials of the premix are dispersed in the Aroclor and then the following materials are added and mixed:

| Added materials: | Parts by weight: |
|---|---|
| Monsanto Aroclor 1254 | 55 |
| Neville Chem. Co. 10° Nevillac | 25 |

The premix is added to the rubber solution and mixed. After the mixture is thoroughly dry, it is then cured for 30 minutes at 250 °F.

Whatever its composition, it is preferable that the thickness of the adhesive composition is from about 0.003 inch to 0.017 inch, more preferably about 0.005 inch to 0.010 inch, for most applications.

## Claims

1. A resealable coupling device for use in medical applications, the device including a pair of first and second coupling members (4, 6), said first coupling member (4) having a first opposed surface for releasably engaging a second opposed surface of the second coupling member to form a releasable fluid-tight seal therewith,
**characterized in that**
at least one of respective opposed surfaces of said first (4) and second (6) coupling members having thereon a single layer of a water-washable adhesive operable to provide releasable and resealable engagement of said first and second coupling members.

2. A device according to Claim 1, wherein said adhesive layer is included on each of said opposed surfaces.

3. A device according to Claim 1, wherein the adhesive includes at least one thermoplastic elastomer.

4. A device according to Claim 3, wherein the thermoplastic elastomer is a styrene copolymer or an acrylic resin.

5. A device according to Claim 4, wherein the styrene copolymer is selected from the group consisting of strene-acrylonitrile-butadiene copolymer having a relatively high butadiene content.

6. A device according to Claim 5, wherein the styrene copolymer is a styrene-isoprene-styrene copolymer or a styrene-acrylonitrile-butadiene copolymer having a relatively high butadiene content.

7. A device according to Claim 5, wherein the styrene copolymer is combined with an oil to form a gel.

8. A device according to Claim 1, wherein the adhesive comprises an elastomeric composition having a modulus of elastic sufficient to enable waste particles to be readily removed therefrom, and having an internal viscosity low enough to enable water-washing an high enough to provide tack.

9. A device according to Claim 8, wherein the modulus of elasticity is in a range of about 1 to 100 psi and the internal viscosity is in a range of about 1000 to 20, 000 poises.

10. A device according to Claim 9, wherein the adhesive further comprises a plasticizer.

11. A device according to Claim 9, wherein the adhesive is solid, hydrophobic and insoluble in water.

12. A device according to Claim 9, wherein the adhesive comprises high molecular weight polyvinyl chloride.

13. A device according to Claim 1, wherein the adhesive comprises a copolymer of vinyl chloride and vinyl acetate.

14. A device according to Claim 1, in which the single layer of the washable adhesive is on one of said single opposed second surfaces.

15. A device according to Claim 1, wherein at least one of the opposed surfaces has an irregular surface to facilitate anchoring of the adhesive.

16. A device according to Claim 1, wherein at least one of the opposed surfaces is comprised of a film or web.

17. A device according to Claim 16, wherein said film or web is made of a non-woven fabric.

18. A device according to Claim 17, wherein the non-woven fabric is made of a material selected from a group consisting of polypropylene, polyethylene, polyester, rayon and blends thereof.

19. A method of releasably coupling first and second components of a medical device together, **characterized in that** the method includes the step of:
reversibly engaging the first and second components through a single layer of a water-washable adhesive to provide a passageway for the flow of fluid through the device.

20. A method according to Claim 19, wherein the adhesive includes at least one thermoplastic polymer.

21. A method according to Claim 20, wherein the thermoplastic elastomer is a styrene copolymer or an acrylic resin.

22. A method according to Claim 21, wherein the styrene copolymer is selected from a group consisting of styrene-acrylonitrile-styrene, styrene-butadiene-styrene, styrene-isoprene-styrene and styrene-ethylene/butylene-styrene.

23. A method according to Claim 19, wherein the adhesive has a modulus of elasticity sufficient to enable waste particles to be removed therefrom and has an internal viscosity low enough to enable water-washing and high enough to provide tack.

24. A method according to Claim 23, wherein the modulus of elasticity is in a range of 1 to 100 psi and the internal viscosity is in a range of about 1000 to 20, 000 poises.

25. A method according to Claim 19, wherein the adhesive further comprises a plasticizer.

26. A method according to Claim 19, wherein the adhesive is solid, hydrophobic and insoluble in water.

27. A method according to Claim 19, wherein the adhesive comprises high molecular weight polyvinyl chloride.

28. A method according to Claim 19, wherein the adhesive comprises a copolymer of vinyl chloride and vinyl acetate.

29. An adhesive for coupling first and second opposing surfaces in a medical device, said adhesive comprising at least one thermoplastic elastomer.

30. An adhesive according to Claim 29, wherein the thermoplastic elastomer is a styrene copolymer or an acrylic resin.

31. An adhesive according to Claim 30, wherein the styrene copolymer is selected from a group consisting of strene-acrylonitrile-butadiene copolymer having a relatively high butadiene content.

32. An adhesive Claim 31, wherein the styrene copolymer is a styrene-isoprene-styrene copolymer or a styrene-acrylonitrile-butadiene copolymer having a relatively high butadiene content.

33. An adhesive according to Claim 31, wherein the styrene copolymer is combined with an oil to form a gel.

34. An adhesive according to Claim 29, wherein the adhesive comprises an elastomeric composition having a modulus of elasticity sufficient to enable waste particles to be readily removed therefrom, and having an internal viscosity low enough to enable water-washing an high enough to provide tack.

35. An adhesive according to Claim 34, wherein the modulus of elasticity is in a range of about 1 to 100 psi and the internal viscosity is in a range of about 1000 to 20, 000 poises.

36. An adhesive according to Claim 35, wherein the adhesive further comprises a plasticizer.

37. An adhesive according to Claim 35, wherein the adhesive is solid, hydrophobic and insoluble in water.

38. An adhesive according to Claim 35, wherein the adhesive comprises high molecular weight polyvinyl chloride.

39. An adhesive according to Claim 29, wherein the adhesive comprises a copolymer of vinyl chloride and vinyl acetate.
